# EUROPEAN PATENT APPLICATION

(11) **EP 2 000 433 A1**
(43) Date of publication of application: **10.12.2008**
(21) Application number: 07252323.6
(22) Date of filing: 08.06.2007
(51) Int. Cl.: C01B 3/22, C07C 51/12, C07C 67/36, C07C 53/08, C07C 69/14

(54) **Process for the preparation of acetic acid and/or methyl acetate**

(71) Applicant: BP Chemicals Limited, Sunbury-on-Thames, Middlesex TW16 7BP (GB)
(72) Inventor: Ditzel, Evert Jan, East Yorkshire, HU12 8DS (GB); Ellis, Brian, East Yorkshire, HU16 4HY (GB); Gaemers, Sander BP Chemicals Limited, Middlesex, TW16 7LN (GB); Kay, Richard Daniel BP Chemicals Limited, Middlesex, TW16 7LN (GB); Morris, George Ernest, East Yorkshire, HU16 4BQ (GB); Sunley, John Glenn, East Yorkshire, HU12 8DS (GB)
(74) Representative: Brooke, Caron

(57) **Abstract**

Process for the production of acetic acid and/or methyl acetate by contacting methanol and/or a reactive derivative thereof with a decomposition catalyst to produce a mixture of carbon monoxide and hydrogen and subsequently catalytically converting the carbon monoxide and hydrogen to acetic acid and/or methyl acetate.

## Description

The present invention relates to the production of carbonylation products, and in particular to a process for the production of acetic acid and/or methyl acetate from methanol and/or a reactive derivative thereof

The manufacture of acetic acid and its derivatives, such as methyl acetate is typically carried out by the homogeneous carbonylation of methanol, or a reactive derivative thereof, with carbon monoxide in the presence of a Group VIII catalyst such as rhodium and/or iridium catalyst, as described, for example in EP-A-0144935, EP-A-0643034 and US 6,211,405. Typically, a halogen containing promoter such as methyl iodide, hydrogen iodide and/or lithium iodide is employed in such homogeneous processes. Heterogeneous carbonylation processes are also known for the production of acetic acid. EP-A-0596632, for example, describes the use of a mordenite zeolite catalyst exchanged with Cu, Ni, Ir, Rh or Co, and EP-A-0353722 describes a gas-phase carbonylation process employing a solid catalyst comprising a polyoxometalate anion in combination with a group VIII element. One of the advantages of the aforementioned gas-phase processes is that corrosive halide-containing promoters are not required.

Conventionally, carbonylation processes employ carbon monoxide as the carbonylating source. Because of difficulties associated with the transport and storage, carbon monoxide is typically generated on site by steam or autothermal reforming of a hydrocarbon, such as natural gas or naphtha. Reforming generates synthesis gas, a mixture of carbon monoxide, hydrogen and carbon dioxide and has to be separated cryogenically to give carbon monoxide of sufficient purity for use in conventional methanol carbonylation processes to produce acetic acid. Carbon monoxide production thereby necessitates high capital and energy costs. It would therefore be desirable if these capital and energy costs could be largely eliminated or reduced. Depending on the location and feedstock availability carbon monoxide production also requires a supply of natural gas or other hydrocarbon such as naphtha. In the absence of a local supply of natural gas imported naphtha is typically used as feedstock, as it is more readily transportable than natural gas. Naphtha can be an expensive feedstock and it would be desirable to replace it with an easily transportable alternative feedstock derived from low cost natural gas in remote locations. One such feedstock is methanol.

WO 01/07393 describes processes for converting synthesis gas to oxygenated products such as esters, acids, acid anhydrides and mixtures thereof and also describes processes for converting an alcohol and/or ether to oxygenated products such as esters, acids, acid anhydrides and mixtures thereof. The reaction conditions for the processes are high temperature and high pressure.

Thus there remains a need for an improved and/or an alternative method for producing acetic acid and/or methyl acetate from methanol and/or a reactive derivative thereof.

Accordingly, the present invention provides a process for the production of at least one of acetic acid and methyl acetate which process comprises the steps of
(i) contacting a feed stream comprising methanol and/or a reactive derivative thereof in a reaction zone with a decomposition catalyst capable of converting the feed stream to an output stream comprising carbon monoxide, hydrogen and optionally methanol; and subsequently
(ii) contacting at least a portion of the output stream of step (i) in a reaction zone with a carbon monoxide conversion catalyst in the absence of a halide-containing promoter to produce a product stream comprising at least one of acetic acid and methyl acetate.

The process of the present invention advantageously allows the generation of carbon monoxide in-situ without the need for an external source of carbon monoxide. Furthermore, the use of a non halide containing promoter in the process of the present invention allows, dimethyl ether, methyl acetate, acetic acid and unreacted methanol to be recycled to step (i) of the process without the need for purification and treatment to remove halides prior to being recycled. More advantageously, the process may be carried out at low pressures.

In step (i) of the process of the present invention there is employed a catalyst which is active for the decomposition of methanol and/or a reactive derivative thereof to carbon monoxide, hydrogen and optionally unconverted methanol. Suitable catalysts include at least one conventional methanol decomposition catalyst such as a supported metal of Group VIII of the Periodic Table either alone or in combination with one or more other metals from Groups I to VIII of the Periodic Table. The Periodic Table referred to is that contained in Cotton and Wilkinson, 4th Edition, published by John Wiley & Sons in 1980. Thus, suitable methanol decomposition catalysts include supported palladium-containing catalysts, supported nickel-containing catalysts, supported rhodium-containing catalysts and supported cobalt-containing catalysts. Each of these catalysts may be used alone or in combination with other metals. The Group VIII metal may be admixed with one or more other Group VIII metals and/or with metals of Groups I to VII of the Periodic Table, such as iron, copper, chromium, gold and zinc. Supports which may suitably be used include oxide supports such as silica, alumina, titania, ceria, zirconia, and zeolites both natural and synthetic and mixtures thereof. Preferred supports include silica, ceria and ceria-zirconia mixtures. Suitably, the methanol decomposition catalyst is a supported rhodium-containing, or a palladium-containing catalyst such as rhodium supported on silica, palladium supported on silica and palladium supported on ceria with or without zirconia.

Other suitable catalysts include those conventionally known as methanol synthesis catalysts. Such catalysts are, for example, described in Kirk-Othmer, Encyclopedia of Chemical Technology, 4th Edition, 1996. Suitable catalysts include supported copper-containing catalysts, zinc-containing catalysts and mixtures thereof. Suitably the support is an oxide support such as alumina, silica, titania, zirconia, chromia and mixtures thereof. Suitable catalysts include copper admixed with zinc oxide supported on alumina, and zinc oxide supported on chromia.

Catalysts suitable for converting methanol or reactive derivatives thereof to a mixture of carbon monoxide, hydrogen and optionally unconverted methanol will hereinafter be referred to as 'decomposition catalysts'

To prevent deactivation of the decomposition catalyst, it is preferable for the feed streams and any recycle streams to be substantially free of inorganic and organic halide compounds such as methyl iodide, hydrogen iodide or lithium iodide. The recycle streams should also be preferably free of C₂ and higher alkyl iodide compounds, such as ethyl and hexyl iodide, which are known to be present in iodide based methanol carbonylation systems but which can deactivate the methanol decomposition catalyst. It is to be understood that any halide fixed to or is otherwise an integral part of the catalyst is permissible in the process of the present invention.

Preferably, in step (i) of the process of the present invention, the extent of decomposition of the methanol and/or reactive derivative to carbon monoxide and hydrogen is at least 50% of the total methanol in the feed (including recycles) to the decomposition catalyst reaction zone.

Optionally, carbon dioxide may be fed to the decomposition catalyst reaction zone to aid decomposition of the methanol/reactive derivative. Suitably, the amount of carbon dioxide employed may be greater than 0 to 10 mol% of the total feed (including recycles).

The carbon monoxide conversion catalyst may be any halide-free heterogeneous catalyst active for the reaction of carbon monoxide with methanol and/or reactive derivative thereof in the absence of a halide-promoter to form acetic acid and/or methyl acetate. These catalysts will hereinafter be referred to as methanol carbonylation catalysts. Suitable methanol carbonylation catalysts are catalysts comprising a solid acid and optionally at least one metal selected from Group VIII and/or Group IB of the Periodic Table of Elements and

The solid acid is any solid material which is acidic. Suitably, the solid acid may be selected from at least one of supported heteropolyacids, zeolites, clays, and acidic oxides such as alumina, silica/alumina and acidic zirconias, for example modified zirconias such as sulphonated or tungstated zirconias. Suitable heteropolyacids include phosphotungstic acids such as H₃PW₁₂O₄₀, phosphomolybdic acids such as H₃PMo₁₂O₄₀, silicotungstic acids such as H₄SiW₁₂O₄₀ and silicomolybdic acids such as H₄SiMo₁₂O₄₀. Suitable zeolites include mordenites, zeolite-beta, zeolite-omega and the ZSM's such as ZSM5. Clays such as pillared clays may be used in the process of the present invention. Specific examples of useful clays are montmorillonite, bentonite and kaolinite. Suitable supports for the solid acid include silica, silica-alumina, zirconia and carbon supports. Suitably, the solid acid is mordenite or a tungstophosphoric heteropolyacid.

Preferably, the metal is selected from Co, Ni, Cu, Ru, Rh, Pd, Ir, Pt and mixtures thereof. More preferably the metal is Rh, Cu and/or Ir.

Examples of suitable methanol carbonylation catalysts are supported catalysts comprising tungstophosphoric acid exchanged with at least one metal from Group VIII and/or Group IB, such as rhodium exchanged tungstophosphoric acid on silica Other suitable catalysts are mordenite and/or ferrierite zeolites. The mordenite and/or ferrierite may be modified by the incorporation of at least one metal from Group VIII and/or Group IB, such as copper and silver. Other examples of suitable methanol carbonylation catalysts are described in WO 01/07393 the contents of which are herein incorporated by reference.

Alternatively, the carbon monoxide conversion catalyst may be any catalyst which is active for the direct conversion of mixtures of carbon monoxide and hydrogen to produce acetic acid. Examples of such catalysts include those based upon a Group VIII metal with optionally one or more additives on an oxide support. The most preferred catalysts are based upon rhodium and ruthenium, most preferably rhodium. Typical additives are selected from one or more of manganese, iridium, scandium, zirconium, hafnium, copper, sodium, potassium and lithium. Such catalysts are described in Progress in C1 Chemistry In Japan, Chapter 6, published by Elsevier in 1989. Typical multicomponent catalysts include those based upon Rh-Mn, Rh-Mn-Ir and Rh-Mn-Ir-Li on a silica support.

The process of the present invention employs two different catalysts. The process may be carried out in a single reactor or in a plurality of reactors, for example 2 or more reactors.

Where a single reactor is employed, the catalysts may be present as a mixture of the catalysts. The reactor may comprise one or more beds of the catalysts mixture. Alternatively, the catalysts may be arranged in sequential beds in a single reactor. Where the catalysts are arranged sequentially, the feed methanol or reactive derivative thereof first contacts the decomposition catalyst to produce an output stream comprising carbon monoxide, hydrogen and unconverted reactant and the output stream is subsequently contacted with a carbon monoxide conversion catalyst which is located downstream of the decomposition catalyst. A single reactor may house a plurality of each of the decomposition and carbon monoxide conversion catalyst beds.

Alternatively, two reactors arranged in series may be employed such that the decomposition catalyst is located in a first reactor and the carbon monoxide conversion catalyst is located in a second reactor downstream of the first reactor. In this configuration, the two reactors may be operated under different reaction conditions. Each reactor may house one or more catalyst beds.

The process of the present invention may be carried out as either a fixed bed or a fluidized bed process or wherein two separate reactors are employed as a combination of a fixed bed and a fluidized bed process.

The reactor may be of any type such as a slurry reactor, a fixed bed reactor, a fluidized bed reactor or where a plurality of reactors is employed, any combination thereof.

The feed for the first step of the process of the present invention is selected from one or more of methanol and/or a reactive derivative thereof. Reactive derivatives of methanol include methyl acetate and dimethyl ether.

In step (i), the feed is converted to a mixture of carbon monoxide, hydrogen and optionally unconverted methanol. Suitably, the molar ratio of H₂ : CO produced is in the range 1 : 1 to 4 : 1, preferably 2 : 1. Where the output stream from step (i) comprises unconverted methanol, the molar ratio of CO : methanol is suitably in the range 0.1 : 1 to 10 : 1, preferably, 5 : 1 to 1 : 5, such as 1 : 1.

A portion of the output stream from step (i) may be recycled to the reaction zone of step (i), together with or separately from the fresh methanol and/or reactive derivative thereof. Alternatively, the components of a portion of the output stream may be separated and subsequently recycled to step (i) reaction zone.

At least a portion of the output stream from the step (i) reaction zone is passed to the reaction zone for step (ii) where it is converted to a product stream comprising at least one of acetic acid and methyl acetate. Other components which may be present in the product stream include unreacted methanol and/or reactive derivative, carbon monoxide, carbon dioxide and hydrogen.

The product stream may be separated into a liquid fraction and a gas fraction using conventional gas/liquid separation equipment such as distillation. The gas fraction may comprise carbon monoxide, carbon dioxide, hydrogen and optionally dimethyl ether and may be recycled to step (i) and/or step (ii). The liquid fraction may comprise acetic acid, methanol, methyl acetate, dimethyl ether, and water and may be separated using any suitable separation equipment such as distillation to recover acetic acid. The methanol, methyl acetate, and dimethyl ether so obtained may be recycled to step (i) and/or step (ii). The acetic acid may, if desired, be further purified using any suitable known method such as distillation.

Depending on the choice of catalyst, carbon dioxide may be produced as a byproduct in either step (i) or step (ii). The carbon dioxide produced in either step may be recycled to step (i).

If a mixture of acetic acid and methyl acetate is produced, at least a portion of the methyl acetate can be hydrolysed by conventional means to produce acetic acid. At least a portion of the methanol obtained from the hydrolysis step may be recycled to step (i) and/or step (ii). Alternatively, at least a portion of the methyl acetate may be recycled to step (i) and/or step (ii).

Unreacted methanol and/or reactive derivative, carbon monoxide and hydrogen in the product stream may be recycled to the step (i) and/or the step (ii) reaction zone.

The feed to the step (i) and/or step (ii) reaction zone may optionally comprise water, preferably as steam. The addition of water can improve catalyst lifetime by reducing the level of coking. It may additionally affect the selectivity of the process, for example, by increasing the yield of acetic acid relative to that of methyl acetate. Where water is employed, the amount of water is suitably 1 to 20 wt% of total feed (including recycles) to step (i) and/or step (ii).

Unreacted water may optionally be separated from the output and/or product streams both reaction zones and/or recycled to the step (i) and/or step (ii) reaction zones.

Suitable temperature and pressure conditions which effect decomposition of methanol to carbon monoxide, hydrogen and optionally unconverted methanol are a temperature in the range 150 to 400 °C, preferably in the range 200 to 350 °C and a pressure in the range 1 to 120 atmospheres, preferably in the range 1 to 50 atmospheres, more preferably in the range 1 to 30 atmospheres.

Suitably, where a methanol carbonylation catalyst is employed in step (ii), the reaction conditions of the step (i) reaction zone are chosen such that at least 50% of the methanol/reactive derivative thereof is decomposed to carbon monoxide and hydrogen. Thus, suitably, the reaction conditions in step (i) are a temperature in the range 150 to 400 °C and a pressure in the range 1 to 30 atmospheres.

Suitable temperature and pressure conditions in step (ii) are a temperature in the range 150 to 400 °C, preferably in the range 200 to 350 °C and a pressure in the range 1 to 120 atmospheres, preferably in the range 1 to 50 atmospheres, more preferably 1 to 30 atmospheres.

Where a fixed bed reactor is used, the total gas hourly space velocity (GHSV) is suitably less than or equal to 10000 h⁻¹ over each catalyst bed, preferably less than or equal to 6000 h⁻¹, and more preferably less than or equal to 5000 h⁻¹.

For a fluid bed reactor suitable fluidization velocities are the range 10 to 90 cm per sec.

Additional gases, such as nitrogen, argon, or volatile hydrocarbons such as methane, ethane or propane may also be present in the feed and/or recycle streams. Alternatively and/or additionally, such gases may be present as diluents.

The invention will now be illustrated by the following non-limiting examples.

### Example 1

### Catalyst Preparation

### Preparation of copper-zinc oxide alumina catalyst

A first solution 'A' of 1M concentration was prepared by dissolving 183.0 g of Cu(NO₃)₂.2.5 H₂O in 500 ml of deionised water at room temperature. A second solution 'B' was prepared by dissolving 74.1 g of Al(NO₃)₃.9H₂O in 500 ml of deionised water at ambient temperature. A third solution 'C' was prepared by dissolving 146.5 g of Zn(NO₃)₂.6H₂O in 500 ml of deionised water at ambient temperature. The three solutions were then mixed (the order of mixing is not deemed critical) at ambient temperature, the resultant solution being clear with a pH of 3.0. The mixed solution was then heated to 85°C, followed by the dropwise addition of a 2M solution of sodium carbonate, until the pH reached 8.0, resulting in the precipitation of a mixed-metal Cu-Zn-Al hydroxy-carbonate catalyst precursor. This precursor was present as a precipitate slurry in water and was blue-turquoise in colour. The catalyst slurry was heated to 85°C for one hour to equilibrate the precipitate composition. On cooling the solution, it was filtered through a Buchner funnel with Whatman filter paper to recover the blue precipitate. During filtration, the filtrate was washed repeatedly with fresh, deionised water until the filtrate solution was clear in colour. The washing process ensured a low content of sodium of < 0.5 % by weight in the precipitate. The filtered, washed solid was then dried at 120°C for 4 hours, followed by calcination at 350°C for 3 hours in static air, resulting in a black solid.

### Preparation of silica supported rhodium exchanged phosphotungstic acid catalyst

1.000 g rhodium acetate was dissolved in 50 ml methanol under nitrogen for 30 minutes. To this solution was added 13.033 g of the heteropolyacid H₃PW₁₂O₄₀, followed by stirring for 4 hours. To this solution was mixed 5.892 g of silica, with the nitrogen flush maintained for 4 hours. The slurry mixture was then placed in a rotary evaporator at 40°C to remove the methanol by evaporation, at a reduced pressure of 100 mbar for 30-60 minutes. The resulting solid was then stored under nitrogen prior to catalyst testing.

### Preparation of Methyl Acetate from Methanol

4.6g (5 mL) of the copper/zinc oxide/alumina catalyst was loaded on top of 2.7g (3 mL) of the silica supported rhodium exchanged phosphotungstic acid catalyst in a tubular fixed-bed reactor of diameter 12 mm. No packing was employed between the catalysts so that the surfaces of the two catalysts were in close contact. A pre-heater bed of carborundum granules was positioned on top of the copper/zinc oxide/alumina catalyst. The catalyst beds were heated at 5 °C per minute to a maximum of 230 °C over a period of approximately 2 hours under a 150mL per minute flow of carbon monoxide and subsequently for 1 hour under a nitrogen flow of 150mL per minute. The nitrogen was then turned off and liquid methanol was fed into the reactor via a syringe pump at a rate of 2mL of liquid methanol per hour to achieve a gas hourly space velocity (GHSV) over the catalyst beds of 138 h⁻¹. The reaction was conducted for 2 hours at 230 °C and at atmospheric pressure. The temperature was increased to 250 °C and the reaction continued for 2 hours. Finally, the temperature was raised to 280 °C and the reaction continued for a further 2 hours. Whilst feeding methanol the exhaust gas downstream of the reactor was sampled at each temperature and analysed by a gas chromatograph (GC) fitted with TCD and Poropack QS column. The results are shown in Table 1.

**Table 1**

| | | | |
|---|---|---|---|
| **Temperature °C** | 230 | 250 | 280 |
| **Selectivity to Methyl acetate (mol%)** | 50.7 | 49.1 | 56.2 |
| **STY Methyl Acetate g/kg/h)** | 11.1 | 12.7 | 16.7 |

### Example 2

### Catalyst Preparation

### Copper decomposition catalyst

The copper decomposition catalyst was prepared using co-precipitation. Solutions containing 32.2g of copper (II) acetate (Fluka) dissolved in 100mL water, 12.9g of zinc acetate (Riedel) dissolved in 100mL water, 11.5g of aluminium lactate (Riedel) dissolved in 100mL water and 2.5g magnesium nitrate (Fluka) dissolved in 20mL water were mixed together. The mixture was stirred and a solid was co-precipitated by the addition of a 2M potassium carbonate solution (ACS Reagent supplied by Aldrich). This solution was added via a burette to the solution containing the metal salts which was at a temperature of 80 °C, until a pH of 9 was reached in the reaction mixture. The resulting precipitate was aged in the mother liquor for 1 hour. The precipitate was filtered, washed three times with water, and then dried for 24 hours at 100 °C. The solid was then ground to a fine powder, sieved to achieve a particle size less than 250 microns, and then calcined in air at 450 °C for 18 hours. The preparation was designed to give ca. 10g of material with the following composition 64 wt% CuO, 24 wt% ZnO, 10 wt% Al₂O₃, and 2 wt% MgO. Prior to use 0.15g of the calcined solid was reduced at 250 °C in a gas mixture comprising 20 mole % hydrogen and 80 mole % argon for 3 hours (total flow of47mL/min).

### Palladium decomposition catalyst

The palladium decomposition catalyst was prepared using co-precipitation. Solutions containing 4.9g cerium (III) nitrate (Aldrich) dissolved in 100mL water and 0.15g palladium (II) nitrate (Aldrich) dissolved in 20mL water were mixed together. The mixture was stirred and a solid co-precipitated by the addition of a 2M ammonium carbonate solution (ACS Reagent supplied by Aldrich). This solution was added via a burette to the solution containing the metal salts, which was at a temperature of 90 °C, until a pH of 10 was reached in the reaction mixture. The resulting precipitate was aged in the mother liquor for 1 hour. The precipitate was filtered, washed three times with water, and then dried for 24 hours at 100 °C. The solid was then ground to a fine powder, sieved to achieve a particle size less than 250 microns and then calcined in air at 450 °C for 3 hours. A target yield was to obtain 2g of catalysts with a composition of 3wt%Pd supported on CeO₂. Prior to use 0.15g of the calcined catalyst was reduced at 450 °C for 1.5 hours in a gas mixture comprising 20 mole % hydrogen and 80 mole % argon (total flow of 47mL/min).

### Copper mordenite catalyst - Cu(55)MOR

H-Mordenite (100 g) with a silica to alumina ratio of 20 (ex Süd Chemie) was weighed into a 500 mL round bottomed flask together with 18.1g of copper (II) nitrate hemipentahydrate (98% ACS) and a stirrer bar. Sufficient deionised water (ca. 100 mL) was then added to the flask until a thick slurry was obtained. The top of the flask was then loosely covered and the flask left to stir overnight. The zeolite was then dried under reduced vacuum using a rotary evaporator before being dried in an oven at 110 °C for 7 hours. The zeolite was then calcined in a muffle oven (oven volume = 18L) under a static atmosphere of air. The temperature was increased from room temperature to 110°C at a ramp rate of 5°C/min and then held at this temperature for 0.5 hours. The temperature was then increased to 550 oC at a ramp rate of 10 °C/min and held at this temperature for 4 hours. The zeolite was then compacted at 12 tonnes in a 33 mm die set using a Specac Press, and then crushed and sieved to a particle size fraction of 500 to 1000 microns. The mordenite had a copper loading of 55 mol % relative to aluminium in the mordenite. Prior to use 0.65g of Cu(55)-MOR was reduced at 100 °C in a flow of hydrogen (20mL/min).

### Preparation of Acetic Acid and Methyl Acetate from Methanol

0.65g Cu(55)-MOR and 0.15g of one of either the Cu or Pd decomposition catalysts were charged to a quartz reactor tube after having been freshly reduced. The catalysts were arranged in either a stacked bed (decomposition catalyst exposed to the reaction mixture first) or a mixed bed arrangement. The catalyst bed was heated to 300 °C under a gas mixture comprising 60 mole % argon and 40 mole % hydrogen at atmospheric pressure at a gas flow rate of 50mL/min. Once at temperature the gas mixture was changed to one comprising 39 mole % methanol and 61 mole % argon at a gas flow rate of 52.5 mL/min. Condensible products from the reactor were collected by passing the gas from the reactor through a knock out pot (ice bath containing potassium chloride). Acetic acid and methyl acetate STYs were calculated from an offline GC analysis (Thermo Quest model KA00 gas chromatograph and HP-Plot Q capillary column) of the liquid collected in knock out pot at the end of 5 hour reaction runs. The reactions were repeated so that an estimate of the error could be calculated. The GHSV was 2625 h⁻¹ (calculated with respect to the Cu(55)-MOR catalyst). The results are given in Table 2.

**Table 2**

| **Decomposition** | **Conversion** | **Reaction** | **Bed** | **STY Acetic** | **STY Methyl** |
|---|---|---|---|---|---|
| **Catalyst** | **Catalyst** | **Temp.** | **Configuration** | **Acid** | **acetate** |
| | | **°C** | | **(g/kgcat/h)** | **(g/kgcat/h)** |
| Palladium | Cu(55)-MOR | 300 | Stacked | 9.85 ± 0.45 | 0.75 ± 0.35 |
| Palladium | Cu(55)-MOR | 300 | Mixed | 1.45 ± 0.05 | 0.3 |
| Copper | Cu(55)-MOR | 300 | Stacked | 6.5 ± 0.6 | 0.4 |
| Copper | Cu(55)-MOR | 300 | Mixed | 0.3 ± 0.1 | 0.07 |

## Claims

1. A process for the production of at least one of acetic acid and methyl acetate which process comprises the steps of
(i) contacting a feed stream comprising methanol and/or a reactive derivative thereof in a reaction zone with a decomposition catalyst capable of converting the feed stream to an output stream comprising carbon monoxide, hydrogen and optionally methanol; and subsequently
(ii) contacting at least a portion of the output stream of step (i) in a reaction zone with a carbon monoxide conversion catalyst in the absence of a halide-containing promoter to produce a product stream comprising at least one of acetic acid and methyl acetate.

2. A process according to claim 1 wherein the feed stream comprises methanol

3. A process according to claim 1 wherein the reactive derivative is selected from methyl acetate and dimethyl ether.

4. A process according to claim 1 or claim 2 wherein the decomposition catalyst comprises a supported metal of Group VIII of the Periodic Table.

5. A process according to claim 4 wherein the supported metal is selected from palladium, nickel, rhodium and cobalt.

6. A process according to claim 4 or claim 5 wherein the catalyst also comprises one or more metals from Groups I to VII of the Periodic Table.

7. A process according to claim 6 wherein the metal is selected from iron, copper, chromium, gold and zinc.

8. A process according to any one of claims 4 to 7 wherein the support is an oxide support or a zeolite.

9. A process according to claim 8 wherein the oxide support is selected from alumina, silica, titania, ceria, zirconia and mixtures thereof.

10. A process according to claim 1 wherein the decomposition catalyst is selected from rhodium supported on silica, copper and zinc supported on alumina, palladium supported on ceria, palladium supported on silica, zinc oxide supported on chromia and palladium supported on a mixture of ceria and titania.

11. A process according to any one of claims 1 to 10 wherein the feedstream is substantially free of inorganic and organic halide compounds.

12. A process according to any one of claims 1 to 1 wherein at least 50% of the total methanol in the feedstream (including recycle streams) is decomposed to carbon monoxide and hydrogen.

13. A process according to any one of claims 1 to 12 wherein carbon dioxide is also fed to the reaction zone in step (i).

14. A process according to claim 13 wherein the carbon dioxide is fed in an amount of greater than 0 to 10 mol% of the total feed to step (i) including recycles.

15. A process according to any one of the preceding claims wherein the carbonylation catalyst is a solid acid catalyst.

16. A process according to claim 15 wherein the solid acid catalyst is selected from a supported heteropolyacid, zeolites, clays and acidic oxides.

17. A process according to claim 17 wherein the supported heteropolyacid is selected from phosphotungstic acids, phosphomolybdic acids, silicotungstic acids and silicomolybdic acids.

18. A process according to claim 17 wherein the heteropolyacid comprises a metal selected from Co, Ni, Cu, Ru, Rh, Pd, Ir, Pt and mixtures thereof.

19. A process according to claim 18 wherein the metal is selected from Rh, Cu, Ir and mixtures thereof.

20. A process according to claim 16 wherein the zeolite is selected from mordenite, ferrierite, a ZSM type zeolite, zeolite-beta and zeolite-omega.

21. A process according to claim 20 wherein the zeolite is a mordenite and/or a ferrierite.

22. A process according to claim 21 wherein the mordenite and/or ferrierite comprises a Group IB metal.

23. A process according to claim 22 wherein the Group IB metal is Cu or Ag.

24. A process according to claim 16 wherein the clay is selected from montmorillonite, bentonite and kaolinite.

25. A process according to claim 1 wherein the conversion catalyst is a Group VIII metal supported on an oxide support.

26. A process according to claim 25 wherein the Group VIII metal is ruthenium or rhodium.

27. A process according to claim 25 or claim 26 wherein the conversion catalyst further comprises at least one of manganese, iridium, scandium, zirconium, hafnium, copper, sodium, potassium and lithium.

28. A process according to claim 25 wherein the conversion catalyst comprises rhodium and manganese supported on silica.

29. A process according to any one of claims 1 to 28 wherein the catalysts of steps (i) and (ii) are in a single reactor.

30. A process according to claim 29 wherein the catalysts are present in the reactor as one or more beds of a mixture of the catalysts.

31. A process according to claim 29 wherein the catalysts are present in the reactor as separate beds of catalyst.

32. A process according to claim 29 wherein the reactor comprises a plurality of separate catalyst beds.

33. A process according to any one of claims 1 to claim 28 wherein the decomposition catalyst and the conversion catalyst are in separate reactors.

34. A process according to any one of the preceding claims wherein the process is carried out as a fixed bed or fluidised bed process.

35. A process according to any one of the preceding claims wherein the output stream of step (i) comprises hydrogen and carbon monoxide in a molar ratio in the range 0.1 : 1 to 10 : 1.

36. A process according to claim 35 wherein the molar ratio is in the range 5 : 1 to 1:5.

37. A process according to claim 36 wherein the molar ratio is 1 : 1.

38. A process according to any one of the preceding claims wherein at least a portion of the output stream from step (i) is recycled to the reaction zone of step (i).

39. A process according to any one of the preceding claims wherein carbon dioxide is produced in step (i) and/or step (ii).

40. A process according to claim 39 wherein the carbon dioxide is recycled to step (i).

41. A process according to any one of the preceding claims wherein the product stream of step (ii) is separated into a liquid fraction and a gas fraction.

42. A process according to any one of the preceding claims wherein the product stream comprises at least one of acetic acid and methyl acetate.

43. A process according to claim 42 wherein at least a portion of the methyl acetate is hydrolysed to acetic acid.

44. A process according to claim 42 wherein at least a portion of the methyl acetate is recycled to step (i) and/or step (ii)

45. A process according to any one of the preceding claims wherein water is also fed to the reaction zones of step (i) and/or step (ii).

46. A process according to claim 45 wherein the water is fed in an amount in the range 1 to 20 wt% based on total feed (including recycles).

47. A process according to any one of the preceding claims wherein step (i) and/or step (ii) is carried out at a temperature in the range 150 to 400 °C and at a pressure in the range 1 to 120 atmospheres.

48. A process according to claim 47 wherein step (i) is carried out at a temperature in the range 150 to 400 °C and at a pressure in the range 1 to 30 atmospheres.

49. A process according to claim 47 wherein step (ii) is carried out at a temperature in the range 200 to 350 °C and at a pressure in the range 1 to 50 atmospheres.

50. A process according to claim 1 wherein the process is operated as a fixed bed process, the gas hourly space velocity over each catalyst is less than or equal to 10000 h⁻¹.

51. A process according to claim 50 wherein the gas hourly space velocity over each catalyst is less than or equal to 5000 h⁻¹

52. A process according to claim 1 wherein the process is operated as a fluid bed process and the fluidisation velocity is in the range 10 to 90 cm s⁻¹.
